# EUROPEAN PATENT APPLICATION

(11) **EP 2 168 627 A1**
(43) Date of publication of application: **31.03.2010**
(21) Application number: 08164178.9
(22) Date of filing: 11.09.2008
(51) Int. Cl.: A61M 25/06

(54) **Safety I.V. catheter with cylindrical tip protector**

(71) Applicant: Eastern Medikit Ltd., 110 007 Delhi (IN)
(72) Inventor: Narang, Karun Raj, 110 007 Delhi (IN); Paul, Sanjeev, 110 007 Delhi (IN)
(74) Representative: Harrison, Robert John

(57) **Abstract**

This invention relates to a medical safety intravenous (I.V.) catheter and in particular, a safety intravenous (I.V.) catheter device with a cylindrical tip protector. The Cylindrical Tip Protector slides to the butterfly portion at the time of cannulation and the needle run its length between the Cylindrical Tip Protector and the needle hub and wherein the forming on the needle gets locked inside the cylindrical portion by means of stainless clip contained in the hollow of the cylindrical portion.

## Description

The following specification particularly describes the nature of this invention and the manner in which it is to be performed:

This invention relates to a safety intravenous (I.V.) Catheter and, in particular, to a safety intravenous (I.V.) Catheter device with retractable needle. In particular, the intravenous catheter device for the invention has been provided to reduce needle stick injuries, spread of hospital acquired infections and also avoid environmental concerns / hazards in its use and disposal. Importantly also apart from being safe and user friendly the intravenous catheter device is cost effective to favour wide scale application and use of the catheter device.

### BACKGROUND ART

It is well known that in health care, one of the most relevant concerns relate to provision of safe hospital environment, in particular, members of hospital and medical staff are required to be assured that they are not subject to any unwanted infection while caring for patients. Such adequate care and concern for safe medical attention and treatment is important not only in hospitals but also for any health care environments such as those required in nursing homes, health homes and related health care facilities and provisions.

Intravenous (I.V.) Catheters are well known medical devices used to obtain continuous vascular access in patients. Such a device generally consists of a hollow-bore needle and an over-the-needle plastic tubing used to access the lumen of a blood vessel of a patient. The intravenous (I.V.) Catheter for such purpose is advanced into the vessel and is used in administering intravenous fluids, medications or blood products. Importantly, in such application and use since the (I.V.) Catheter is placed percutaneously, the hollow-bore needle stylet becomes blood contaminated and when the blood vessel lumen is accessed, the needle-stylet becomes blood -filled.

In the conventional Catheter units presently in use, the basic steps followed in catheter application/use include (i) using alcohol wipes (ii) puncturing skin using needle tip enclosed in a thin plastic tube (iii) moving needle tip forward to puncture vein (iv) blood flashback in the blood collection chamber (v) initiating withdrawal of the needle out from the cannula and leaving the tubing inside the body (vi) The butterfly portion attached to the tubing is fixed by tape to the body of the patient and medicines etc. are given through the opening provided in this portion. (vii) discarding needle with the tip remaining exposed in the sharp's container. Thus the exposed needle remains a potential source for contamination and infection. Infection from such needle stick injuries is a greatest risk for the medical and paramedical staff.

Needle stick injuries from intravenous (I.V.) Catheter stylets are in the high risk category for potential transmission of blood borne pathogens to the injured health care worker, since the same are hollow bore needles which are usually filled with undiluted blood. The blood borne pathogens of greatest concerns include human immuno deficiency virus (HIV), the etiologic agent of the acquired immuno deficiency syndrome (AIDS), hepatitis B virus and hepatitis C virus.

As per hospital reports, one-third nurses and laboratory staff suffer from needle stick injuries every year. HIV transmission through needle injuries is 0.3% of all modes of contaminations. The rate of HIV transmission through needle stick injuries is 2-7% of all means. Injuries from used needle or contact with infected blood on the needle pose serious dangers to health care workers. The nurses are most vulnerable to needle stick injuries among health workers. Apart from health care workers, cleaners, laundry workers, porters, ragpickers etc. are also prone to health hazards caused by improper waste disposal methods. Blood exchange through needle stick injuries can pass on over about 20 deadly pathogens and microorganisms. Health staff with significant exposure to HIV is 6-30% risk of getting infected. Atleast one in eight, health care workers is exposed to fatal infection through needle stick injuries.

To avoid the above hazards of needle stick injuries guidelines have also been provided to benefit medical staff, laboratory staff and employees for protection and minimizing the risk of such contamination / transmission of infection through needle. However, it is obvious that the effectiveness of such precautionary guidelines would depend on accountability, education, training and vigilant compliance. Such precautionary guidelines while being important and most certainly required to be followed, negligence and non-compliance of such guidelines can and usually lead to the afore-discussed problems and complexities of needle stick injuries and hospital acquired infection. It is, therefore, important not only to provide the guidelines but also human care systems/devices, which would minimize the risk of such unwanted contamination especially due to human lapses which can take place any moment and lead to injuries / contaminations of even serious nature such as the HIV / HCB transmission.

### OBJECTS OF THE INVENTION

It is thus the basic object of the present invention to provide a safety intravenous (I.V.) Catheter with retractable needle which would favour safe and user friendly application and disposal of a catheter device.

Another object of the present invention is directed to provide a safety (I.V.) Catheter device adapted to reduce needle stick injuries, spread of hospital acquired infection and the like and thereby ensure safety for the staff involved in the hospital and medical care facilities.

Another object of the present invention is directed to provide for an intravenous (I.V.) Catheter device which would favour safety and effective use of (I.V.) Catheter which would not be subject to any high risk in its use and application as a potential vehicle for transmission of blood borne pathogen to the health care workers.

A further object of the present invention is directed to an intravenous (I.V.) Catheter device which would be simple and cost effective to manufacture and would also be safe for wide scale application and use without fear or accompanying hazards of needle stick injuries or spread of hospital acquired infection.

### SUMMARY OF THE INVENTION

Thus according to the basic aspect of the present invention there is provided an intravenous (I.V.) Catheter safety device with retractable needle comprising:
a needle hub capable of holding the needle. The needle is a different from the ordinary needle as the needle is having a forming at a distance from the tip.

Importantly, the intravenous (I.V.) Catheter device comprises a one way valve adapted to facilitate extra medication and prevent back flow. The said one way valve preferably comprises a silicone tube. Also, the said one way valve is provided with cap means incorporating a recessed plug with protective skirt to effectively prevent contamination when the valve is not in use. Advantageously also the said one way valve cap is adapted for resting the finger to facilitate effective two-point grip for cannulation.

In accordance with yet further aspect of the invention, the intravenous (I.V.) Catheter device comprises colour coded pot cap for gauge size identification. Also, preferably, the intravenous (I.V.) Catheter device comprises angled and grooved wings adapted for easy fixation and prevent pistoning and rolling of cannula over the patient's body.

It would be apparent from the above that the above disclosed intravenous (I.V.) Catheter device with retractable needle of the invention is adapted to ensure safe and user friendly application and use of such catheter devices.

The Cylindrical tip protector is fitted between the butterfly portion and the needle hub. The Cylindrical portion is hollow and contains a stainless steel clip provided in a particular stage where the top of the clip is formed in L shape and the tip of the L shape is designed to bend towards the main body of the clip. The 'L' shaped steel strip in the Cylindrical tip protector attached to the portion nearer to the hub.
In particular, the catheter device of the invention apart from serving the basic need of a catheter essentially takes care of problems of needle stick injuries and spread of hospital acquired infection through the needle used for cannulation. Importantly, the above catheter device is adapted such that when the needle is withdrawn out from the butterfly portion, the needle tip gets enclosed within the Cylindrical housing by means of the stainless steel clip and the tip is not able to come out of the cylindrical housing i.e. it will be engaged in the Cylindrical housing by **means** of clip. The Cylindrical housing will disengage itself from the butter fly and form a protective cap over the needle tip.
As also provided by way of invention, the safety (I.V.) Catheter is a safety device adapted to protect the user against needle stick injuries.

Importantly, in accordance with preferred aspect, the safety (I.V.) Catheter includes a one-way valve (Silicon tube) to facilitate extra medication and to prevent back flow. Advantageously, the valve is provided with selectively adapted pot cap incorporating a recessed plug with protective skirt to effectively prevent contamination when the valve is not in use. Moreover the pot cap is also adapted for resting the finger to facilitate effective two-point grip for cannulation. It offers safe and convenient needleless methods of atraumatic administration of medicines.

In accordance with further preferred aspect the intravenous catheter device comprises colour coded pot cap for gauge size identification. Moreover, the provision is made for angled and grooved wings adapted to offer easy fixation and prevent pistoning and rolling cannula over the patient's body. More importantly, the above disclosed intravenous catheter device is adapted to facilitate simple and safe use of catheter without the need for specialized training for such range of safety intravenous (I.V.) Catheter.

The details of the invention, its objects and advantages are explained hereunder in greater detail in relation to non-limiting exemplary illustration as per the following accompanying figures:

### BRIEF DESCRIPTION OF THE ACCOMPANYING FIGURES

Figure 1 illustrates an embodiment of the intravenous (I.V.) catheter with cylindrical tip protector housing.

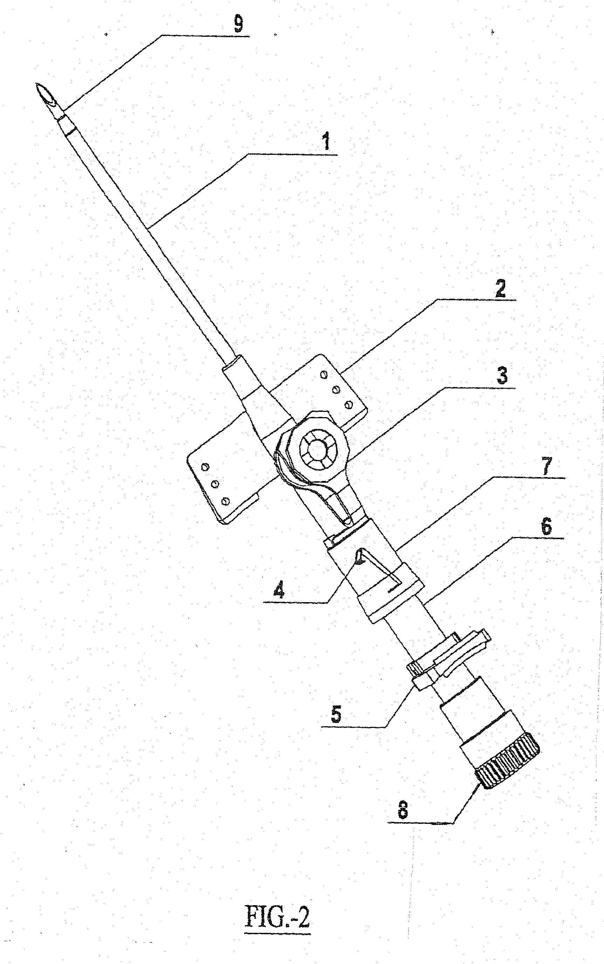

Figure 2 is the complete intravenous (I.V.) catheter wherein the individual parts have been numbered as below:
   1. Portion marked as 1 is plastic tubing.
   2. Portion marked as 2 is butterfly portion.
   3. Portion marked as 3 is Pot cap.
   4. Portion marked as 4 is cylindrical tip protector housing.
   5. Portion marked as 5 is needle hub.
   6. Portion marked as 6 is Stainless Steel clip visible inside the transparent cylindrical housing.
   7. Portion marked as 7 is leur lock.
   8. Portion marked as 8 is needle.

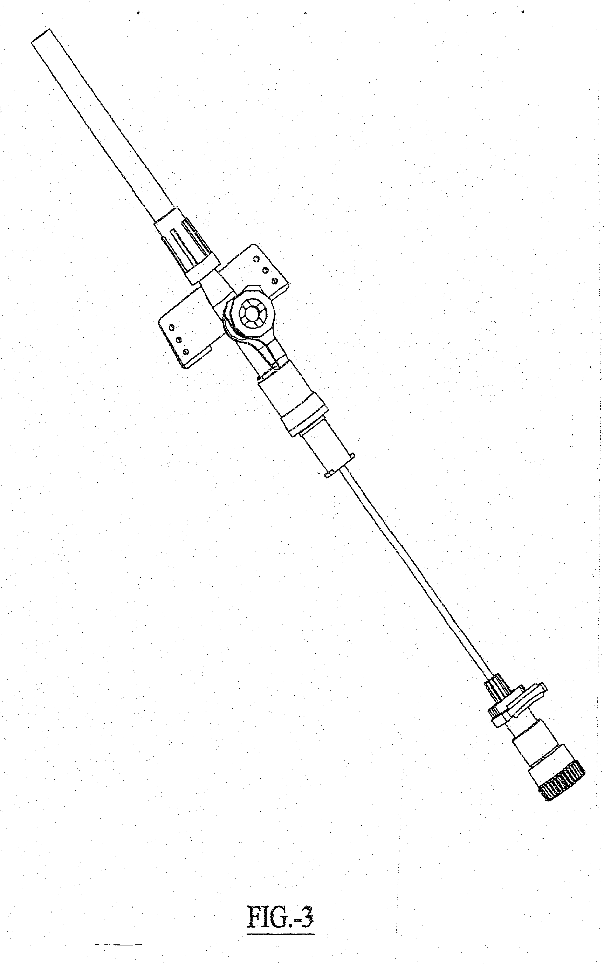

Figure 3 is an illustration of the needle running its full length with tip locked inside the cylindrical housing.

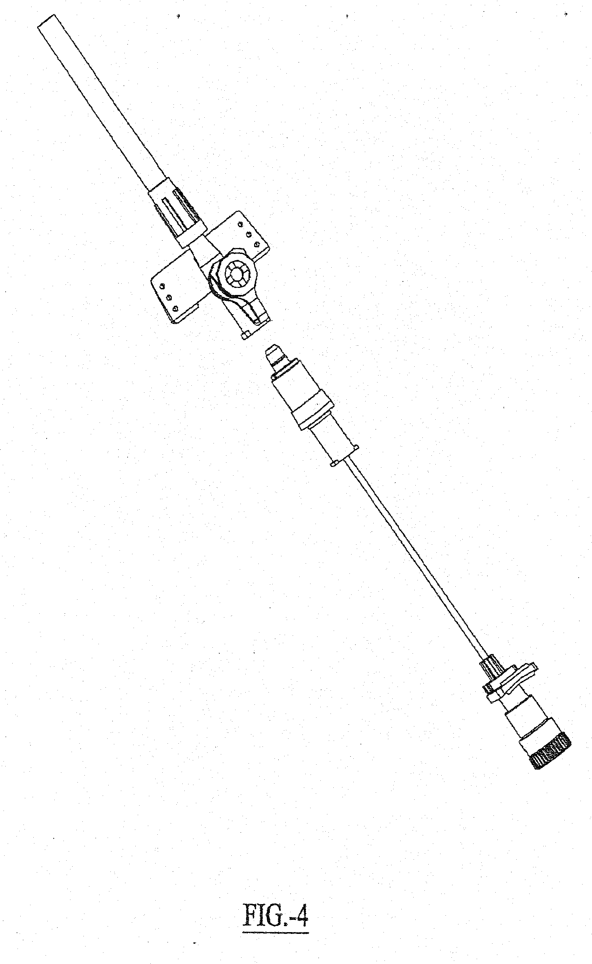

Figure 4 illustrates the intravenous catheter device with the needle retracted within the cylindrical housing, detached from the butterfly portion.

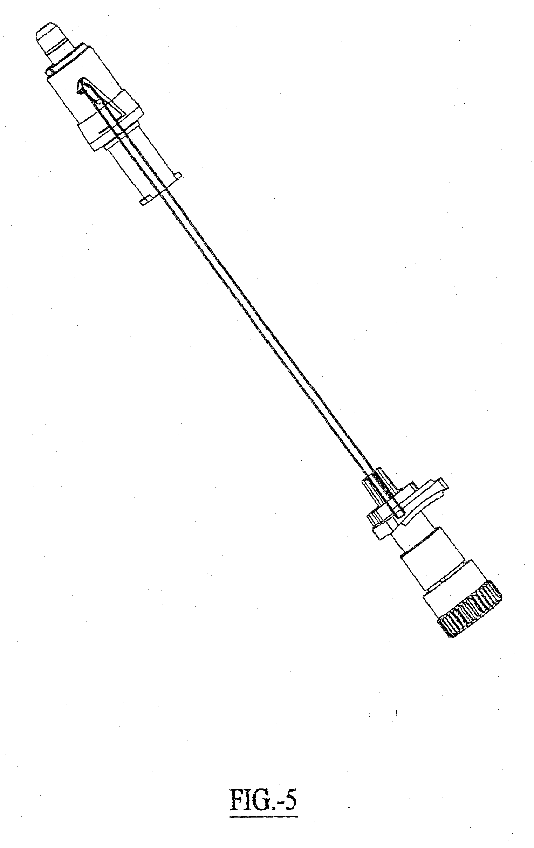

Figure 5 shows the needle tip locked with the help of Stainless Steel clip.

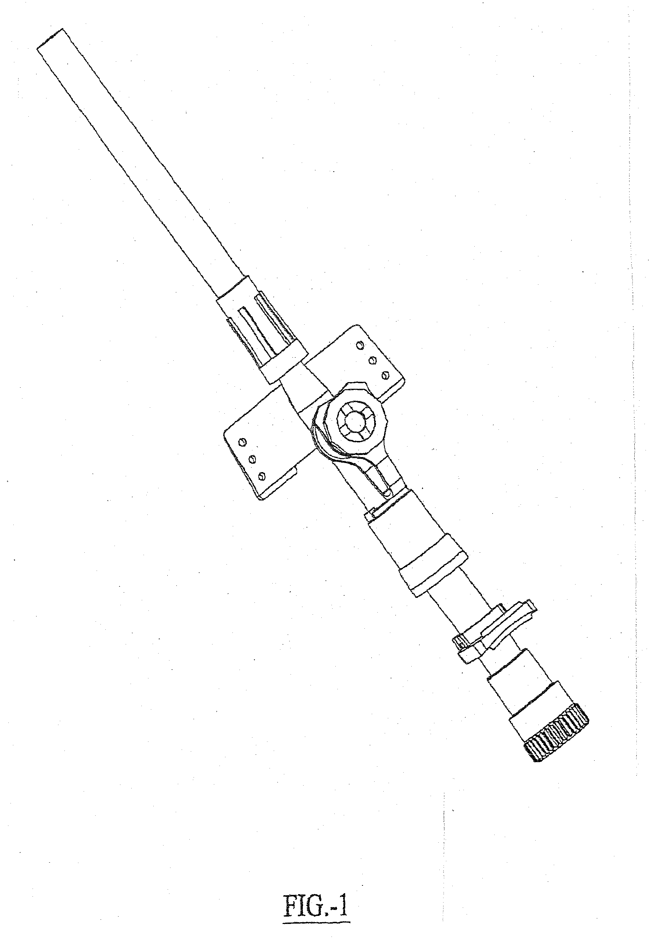

Reference is first invited to accompanying figure 1, which shows the intravenous (i.v.) Catheter device in accordance with the present invention wherein the product I.V. Catheter has a cylindrical tip protector housing between the needle hub and the butterfly portion. The needle is fitted in the needle hub and passes through the housing (and steel stip inside the housing) and the butterfly into the plastic tubing.

Reference is now invited to Figures 3 and 4, which go to illustrate the manner of working of the invention. In figure 3, the housing remains within the butterfly portion and needle runs its full length between the cylindrical housing and the needle hub. In figure 4, the housing is detached from the butterfly portion and the needle tip is locked inside the cylindrical housing tip protector.

Reference is now invited to accompanying figure 5.

Figure 5 shows the needle tip locked within the stainless steel clip and the tip is not able to come out of the cylindrical projection i.e. it will be engaged between the cylindrical projection and the clip.
Figure 5 also shows a rib on the top portion of the cylindrical projection. The purpose of the rib is that it locks the cylindrical projection inside the butterfly. The narrow portion of the cylindrical projection is fitted inside the butterfly in the final assembly.

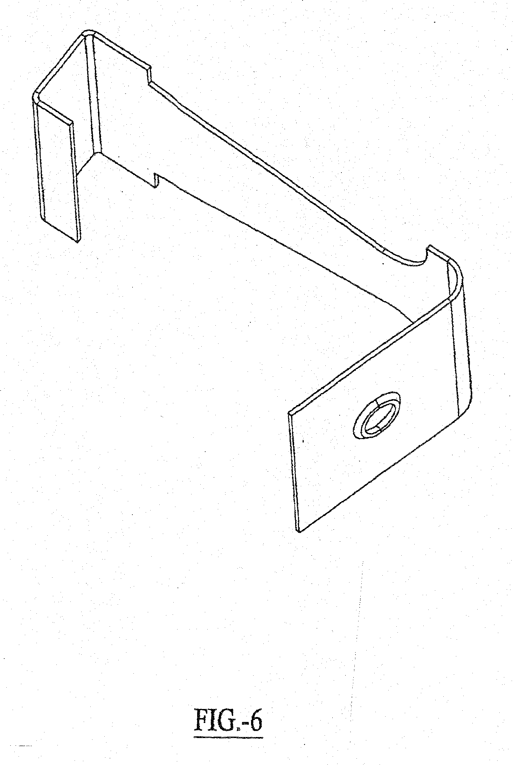

Figure 6 shows the 'L' shaped clip.
The SS clip is in form of L shape having hole on one side and 'U' bend on the other side to accommodate the needle tip. The needle glides through the hole and the 'U' portion acts as a spring and come in front of the needle to lock the needle tip.
Such a provision of the intravenous (I.V.) catheter device therefore achieves the required safety in IV cannulation and adapted to protect the user against needle stick injuries.

The method of application and use of the above safety (I.V.) Catheter of the invention is further detailed hereunder:-
Thus in use of the catheter device initially the position of the catheter insertion is cleaned using alcohol wipes. Thereafter, the skin is punctured by using the needle tip which is inside a thin plastic tubiage. The needle tip is moved forward to puncture the vein. Immediately blood flash back is noticed in the blood collection chamber.

Subsequently, the needle withdrawing action is initiated by withdrawing the needle out from the cannula and leaving the plastic tubing inside the body. The butterfly portion of the catheter remains outside for giving medication. In this process, the cylindrical tip protector slides from its resting place with the narrow portion of the cylindrical tip protector fitted inside the butterfly. In this position, the needle run its full length between the cylindrical tip protector and the needle hub. The needle tip then gets locked inside the cylindrical tip protector by means of stainless steel clip.
As would be apparent from the above, the intravenous (I.V.) safety catheter thus achieves the locking of the needle tip within the tip protector housing free of any exposure to avoid needle stick injury and / or any spread of hospital acquired infection by way of unwanted needle contamination. Finally, by way of the above safe and convenient locking of the needle within tip protective housing of the catheter system of the invention, it is possible to safely discard the needle with cylindrical tip protector in the sharps container.

Advantageously, the above (I.V.) Catheter is adapted to function as safe device to protect the user against needle stick injuries. The above safe catheter device involving the cylindrical tip protector can be obtained of simple materials including polypropylene, low density poly ethylene, stainless steel, acronytrle butadiene styrene, silicon rubber, Floropolymer/ Poly urathene.

The cylindrical tip protector, needle hub, flash back, needle hub are made of polypropylene. The needle and clip are made of stainless steel and pot cap is made of low density polyethylene.

The one way valve can be obtained of silicon tube and adapted to facilitate extra medication and prevent back flow. The valve is specifically adapted to incorporate a recessed plug with protective skirt to effectively prevent contamination when the valve is not in use. Advantageously also pot cap is adapted for resting the finger to facilitate effective two-point grip for cannulation. The system offers safe and convenient needle less method for atraumatic administration of medicines. Moreover, it is possible to provide color coded pot cap for gauge size identification. Angled and groove wings can be provided to offer easy fixation and prevent positioning and rolling of cannula over the patient's body. The catheter device so introduced to this invention is user friendly and provide a safe and simple catheter device for doctors and paramedical staff.

## Claims

1. An intravenous (I.V.) Catheter device with retractable needle comprising:
a needle hub, cylindrical tip protector fitted between the needle hub and butterfly. A needle different from others where a forming is provided at a distance from the tip, where the cylindrical tip protector slides to the butterfly portion and the needle runs its length between the cylindrical tip protector and the butterfly portion and the needle tip forming gets locked in the cylindrical tip protector by the means of a stainless steel clip provided in the hollow portion of the cylindrical tip protector.

2. An intravenous (I. V.) Catheter device as claimed in claim 1 wherein the needle hub is accompanied with a cylindrical tip protector.

3. An intravenous catheter as claimed in claim 1 wherein the cylindrical tip protector is fitted between the needle hub and the butterfly.

4. The intravenous (I.V.) catheter as claimed in claim 1 wherein the narrow portion of the cylindrical tip protector is having ribs which get locked with the butterfly.

5. An intravenous catheter as claimed in claim 1 wherein the cylindrical tip protector slides to the butterfly portion and the needle runs its length between the tip protective housing and the needle hub.

6. An intravenous catheter as claimed in claim 1 where the hollow of the cylindrical tip protector contains a stainless steel clip inside the cylindrical portion.

7. An intravenous (I.V.) Catheter device as claimed in claim 1 where the needle tip forming gets locked in the cylindrical portion and the stainless steel clip provided in the hollow of the cylindrical tip protector.

8. An intravenous (I.V.) catheter device as claimed in class 1 wherein the SS clip is in form of L shape.

9. An intravenous (I.V.) catheter device as claimed in claim 1 wherein the SS clip is having hole on one side and 'U' bend on the other side to accommodate the needle tip.

10. An intravenous (I.V.) catheter device as claimed in claim 1 wherein the needle glides through the hole and 'U' portion act as a spring and infront of the needle to lock the needle tip.

11. An intravenous (I.V.) Catheter device as claimed in claim 1 wherein the needle has a forming at a distance from the tip.

12. An intravenous (I.V.) Catheter device as claimed in claim 1 wherein said one way valve comprises a silicone tube.

13. An intravenous (I. V.) Catheter device as claimed in claim 1 wherein said one way valve is provided with cap means incorporating a recessed plug with protective skirt to effectively prevent contamination when the valve is not in use.

14. An intravenous (I.V.) Catheter device as claimed in claim 1 wherein said one way pot cap is adapted for resting the finger to facilitate effective two-point grip for cannulation.

15. An intravenous (I.V.) Catheter device as claimed in anyone of claims 1 to 7 comprising colour coded pot cap for gauge size identification.

16. An intravenous (I.V.) Catheter device as claimed in anyone of claims 1 to 7 comprising angled and grooved wings adapted for easy fixation and prevent pistoning and rolling of cannula over the patient's body.

17. An intravenous (I.V.) Catheter device with needle substantially as herein described and illustrated with reference to the accompanying figures.
